# EUROPÄISCHE PATENTANMELDUNG

(11) **EP 1 523 947 A1**
(43) Veröffentlichungstag der Anmeldung: **20.04.2005**
(21) Anmeldenummer: 03023356.3
(22) Anmeldetag: 16.10.2003
(51) Int. Cl.: A61B 17/24, A61B 10/00

(54) **Universelles Verfahren und Vorrichtung zur in vivo Simulation und anschliessender in vitro Analyse von biochemischen Körperreaktionen an Gewebeschichten**

(71) Anmelder: DST Diagnostic Science & Technology GmbH, 19061 Schwerin (DE)
(72) Erfinder: Schwertner, Heiko, Dr., 19055 Schwerin (DE)

(57) **Zusammenfassung**

**2.1 Zielsetzung**
   Ziel der in dieser Patentschrift vorgestellten Erfindung ist es, die bestehenden Nachteile der zurzeit vorherrschenden Verfahren und Vorrichtungen für Probennahme und Applikation zu vermeiden. Ferner ein Testverfahren zur teilweisen Simulation von Reaktionskaskaden aufzubauen, mit dem es möglich ist in vivo Reaktion in vitro Messen zu können.
**2.2 Lösung des Problems**
   Entsprechend der Erfindung wird das Problem durch einen Trägerfläche mit einer Kopplungsfläche auf welcher kovalent Substanzen oder Gemische gebunden sind, die durch eine poröse Gaze oder anderer Befestigung am Trägerfläche, und anschließenden Verfahren welche durch die Kontaktierung an der Nasenschleimhaut gelöst. Die Gesamtheit aus Aufbauweise des Trägerfläche mit Kopplungsfläche und Gaze und Art der Ausgestaltung der Substanzen welche an der Kopplungsfläche gebunden sind ist der Simulator, welcher Teile von Reaktionskaskaden von Erkrankungsgeschehen in vivo nachbilden kann, so dass eine ex vivo also in vitro Bestimmung von Testparametern mit dem Stand der Technik entsprechenden analytischen Methoden möglich ist.
**2.3 Anwendungsgebiete**
   Die erfindungsgemäße Anwendung des Simulators am Menschen und Tier sind pharmakologische Testungen, Bestimmung von spezifischen Allergenen, Nachweis von Drogen oder Messung von klinisch relevanten Parametern.

**Anhänge**

**Verzeichnis der Zeichnungen**
   1 Fig. 1 - Beispiel einer anatomisch geformten Klammer mit zwei Trägerflächeflächen (schematisch)
   2 Fig. 2 - Beispiel eines Einklammersystems mit einem oder zwei Trägerfläche für einen Nasenflügel (schematisch)
   3 Fig. 3 - Beispiel eines Einklammersystems mit magnetischer Halterung für einen Nasenflügel (schematisch)
   4 Fig. 4 - Beispiel für Kopplungsflächen mit einer oder mehreren voneinander getrennten oder vermischten Stoffen, Substanzen oder Gemische gebunden auf einer Trägerfläche
   5 Fig. 5 - Gesamtheit aus Trägerfläche, Distanzstück, Kopplungsfläche, Gaze
**Verzeichnis der Literatur**
   1 US 4,800,896 vom 31.01.1989
   2 US 4,887,611 vom 19.12.1989
   3 F. Marcucci und L. Sensi, A new method for IgE detection in nasal mucosa,
   4 Clinical and Experimental Allergy, 1989 Vol.19, Seite 157 - 162
   5 DE 19609315

## Beschreibung

### Hintergrund der Erfindung

Ethische und moralische Grundsätze verbieten es im Normalfall biochemische Tests direkt am Menschen durchzuführen. Deshalb werden, wenn dies notwendig wird z.B. im Erkrankungsfall oder zu Testzwecken, Proben genommen z.B. Blut, Gewebe, Zellen etc. oder es werden Substanzen wie Medikamente, Antibiotika etc. mit Hilfe von Applikatoren eingebracht bzw. appliziert.

Sinnvoll wäre es, zu mindest einen Teil der Reaktionen des Körpers direkt messen zu können. Damit wäre es möglich auf einen großen Teil der Probennahme oder Applikationen verzichten zu können, welche meist mit großen Aufwand und Schmerzen für den Patienten verbunden ist, wie z.B. bei der lebend Gewebeprobennahme in der Nasenschleimhaut oder bei der Darmmucosa.

### Stand der Technik

Es sind verschiedene Verfahren und Vorrichtungen bekannt, wie Probennehmer z.B. US 4,800,896 vom 31.01.1989 oder Applikatoren wie z.B. US 4,887,611 vom 19.12.1989 mit denen es möglich ist entweder Proben zu entnehmen oder Substanzen zu applizieren. Ferner sind in der wissenschaftlichen Literatur verschiedene Probennehmer oder Applikatoren beschreiben wie z.B. bei F. Marcucci und L. Sensi in Clinical and Experimental Allergy, 1989 Vol.19, Seite 157 - 162 mit dem Titel "A new method for IgE detection in nasal mucosa".

### Vorteile der Erfindung

Die Erfindung ersetzt komplett Probennehmer und Applikatoren. In der erfindungsgemäßen Vorrichtung und Verfahren wird ein Teil einer komplexen biochemischen Reaktion des Körper derart simuliert, dass eine Antwort des Körper auf einen äußeren Reitz in vivo extern also in vitro bestimmt bzw. gemessen werden kann. Deshalb kann mit der erfindungsgemäßen Vorrichtung und Verfahren die Vielzahl der bestehenden analytischen Möglichkeiten wie Hochdruckflüssigkeitschromatograpfie (HPLC), Gaschromatographie (GC) aber auch Enzymimmunoassays (ELISA, RIA, EIA, RAST, EAST etc.) zur Messung der Körperantwort ohne große Änderungen genutzt werden.

Der Simulator wird an der Nasenschleimhaut als Fixierungs- und Aktionsort im Körper des Patienten befestigt und kann, im Vergleich zu bestehenden Probennehmern oder Applikatoren, auf sehr milder und angenehmer Weise die ersten Schritte einer komplexen Aktion und Reaktion nachgebildet. Mit dem erfindungsgemäßen Simulator können am lebenden Organismus eine Vielzahl von Tests durchgeführt werden, ohne den Organismus des Patienten zu schädigen oder beeinträchtigen.

### Ziel der Erfindung

Ziel der in dieser Patentschrift vorgestellten Erfindung ist es, die bestehenden Nachteile der zur Zeit vorherrschenden Verfahren und Vorrichtungen für Probennahme und Applikation zu vermeiden. Ferner ein Testverfahren zur teilweisen Simulation von Reaktionskaskaden aufzubauen, mit dem es möglich ist in vivo Reaktion in vitro Messen zu können.

Ein Nachteil der bestehenden Verfahren zur Probennahme ist es, das in vielen Fällen der Körper verletzt werden muss z.B. bei der Gewebeprobennahme. Dies ist mit Schmerzen und eventuell Blutungen (potentielle Infektionsgefahr für das Personal) verbunden. Bei invasiven Probenentnahmeverfahren sind wiederum nicht alle analytischen Möglichkeit die derzeit vorhanden sind nutzbar. Ferner werden bei der Probenentnahme viele biochemische Parameter verändert, z.B. durch Hormon, Mediator-Ausschüttung oder der Unterbrechung von Signalketten innerhalb von Gewebestrukturen.

Vergleichbar ist diese Situation auch bei den Applikatoren. Dort ist es das Problem, dass Substanzen oder Substanzgemische in den Körper gelangen und dort sowohl gewollte als auch ungewollte Reaktionen auslösen können. Dies ist besonders deutlich bei Medikamenten deren gewünschte Wirkung fast immer mit unerwünschten Nebenwirkungen begleitet ist.

Bei allergischen Erkrankungen möchte der behandelnde Arzt zuerst erfahren gegen welche Allergene der Patient eine allergische Reaktion zeigt. Werden jedoch, wie bei so genannten Provokationstests, die Allergene z.B. in die Nase appliziert, durch Einatmung oder durch Trägerfläche auf denen sich die Allergene befinden, können direkt oder durch Diffusion Allergene in den Körper gelangen. Damit soll die körpereigene Abwehrreaktion, im Falle eines positiven Allergens, bestimmt werden. Ungewollterweise werden möglicherweise aber erst eine Allergenisierung ausgelöst, dass heißt erst durch den Kontakt mit dem Allergen, das im Provokationstest eingesetzt wird, bildet sich eine neue Allergie gegen ebendieses Allergen aus. Ferner wird in der Therapie von Allergien die so genannte Desensibilisierung angewendet, bei der kleinsten Menge von Allergenen dem Patienten verabreicht werden. Z.B. der im Patent DE 19609315 vorgeschlagene nasale Probennehmer könnte eine ungewollte Sensibilisierung verursachen und damit dem Patienten Schaden zufügen.

Aufgabe der Erfindung war es diese Nachteile zu minimieren bzw. auszuschließen und dennoch ein für den Patienten erweitertes analytisch methodisches Spektrum zur Verfügung zu haben. Ferner war es die Aufgabe durch die Simulation eines teils des natürlichen Reaktionsweges, die gewünschten Testparameter in messbare Größen zu überführen zu können, ohne die Körperreaktionen im generellen beeinflussen zu müssen.

### Beschreibung der Erfindung

Die Aufgabe wird erfindungsgemäß durch eine Vorrichtung und Verfahren gelöst, dass es ermöglicht, mittels einer Trägerfläche eine oder mehrere Kopplungsflächen oder Strukturen auf der Nasenschleimhaut für eine gewisse endliche Zeit fixieren zu können. Dabei sind Stoffe, Substanzen oder Gemische chemisch kovalent oder chelatartig auf der Kopplungsfläche gebunden, so dass diese unter physiologischen Bedingungen nicht von der Kopplungsfläche entfernt werden oder diffundieren können. Die Kopplungsfläche mit den gebundenen Stoffen, Substanzen oder Gemischen ist mittels einer weit porigen Gase, welche aus verschiedenen Materialien ausgeführt werden kann, auf dem Trägerfläche befestigt oder ohne Gase durch geeignete Verfahren befestigt, z.B. durch verkleben, verschweißen, klemmen, nageln, nieten etc.

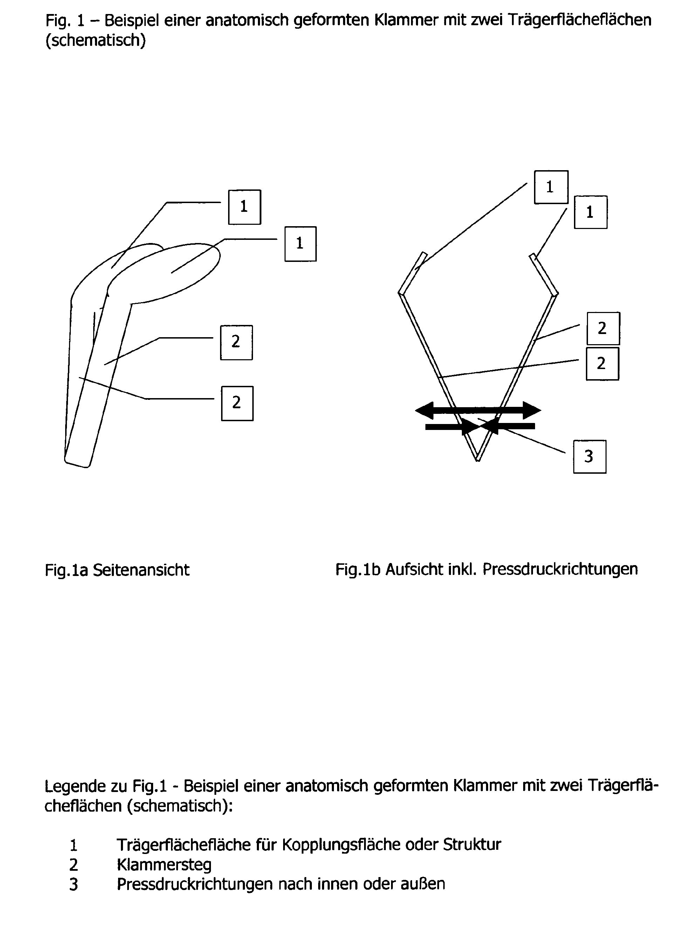

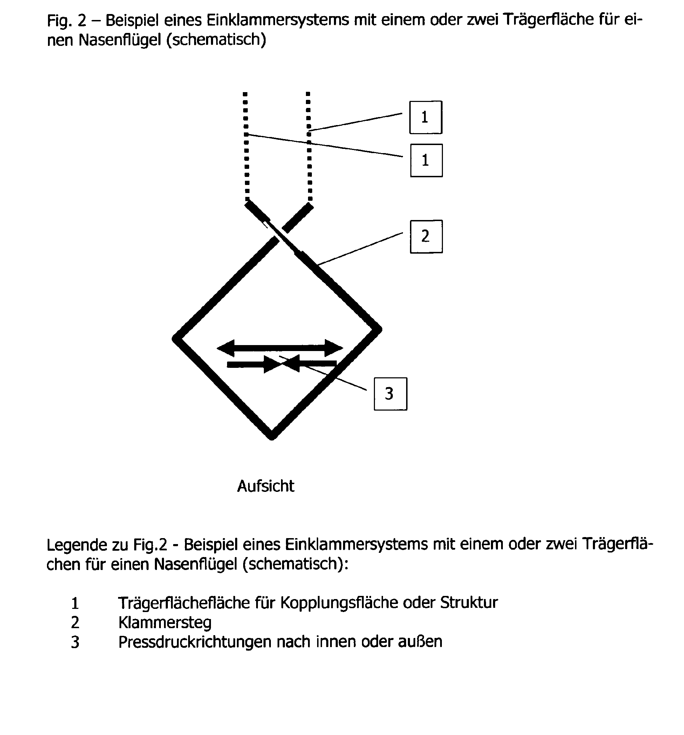

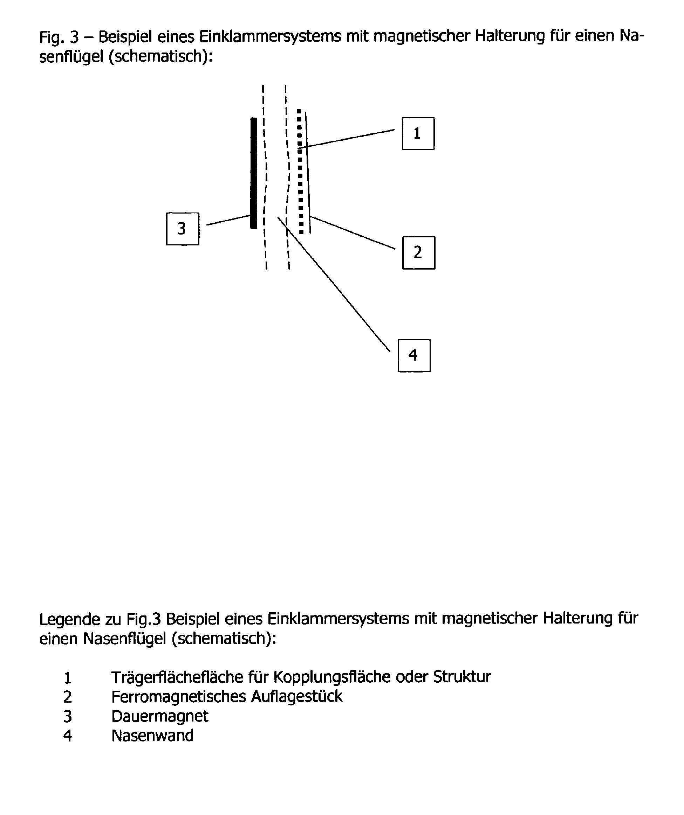

Der Trägerfläche selbst kann als Klammer, die nach außen oder nach innen drückend ist, ausgeführt sein (Fig.1) oder als Einzelklammer für jede Nasenwand (Fig.2 / Fig.3).

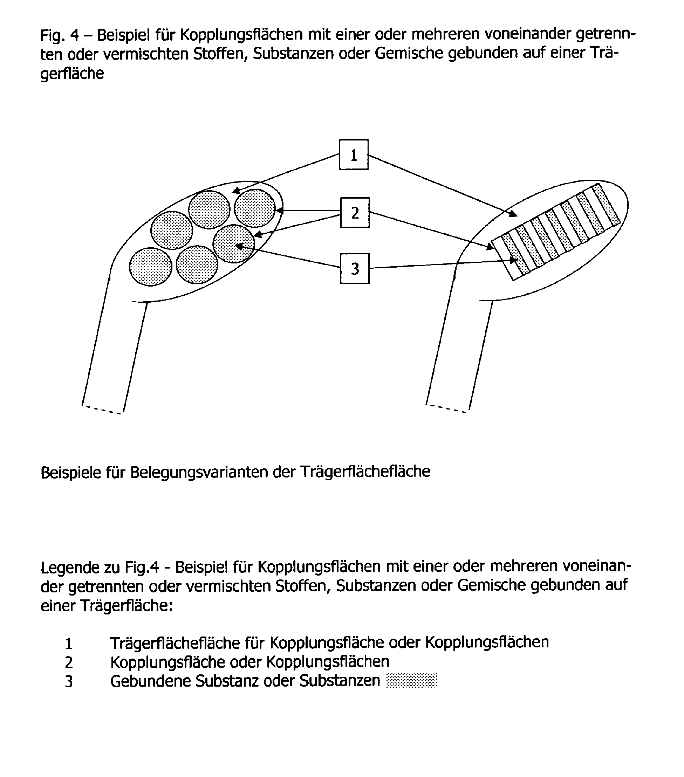

Die Kopplungsfläche oder Struktur auf der die Stoffe, Substanzen oder Gemische gebunden sind kann mehrfach geteilt oder aus einem Stück gefertigt sein. Auf der Kopplungsfläche oder Struktur kann eine oder mehrere von einander getrennte oder vermischte Stoffe, Substanzen oder Gemische gebunden sein oder es können mehrere voneinander getrennte Kopplungsflächen oder Strukturen auf dem Trägerfläche befestigt sein (Fig.4).

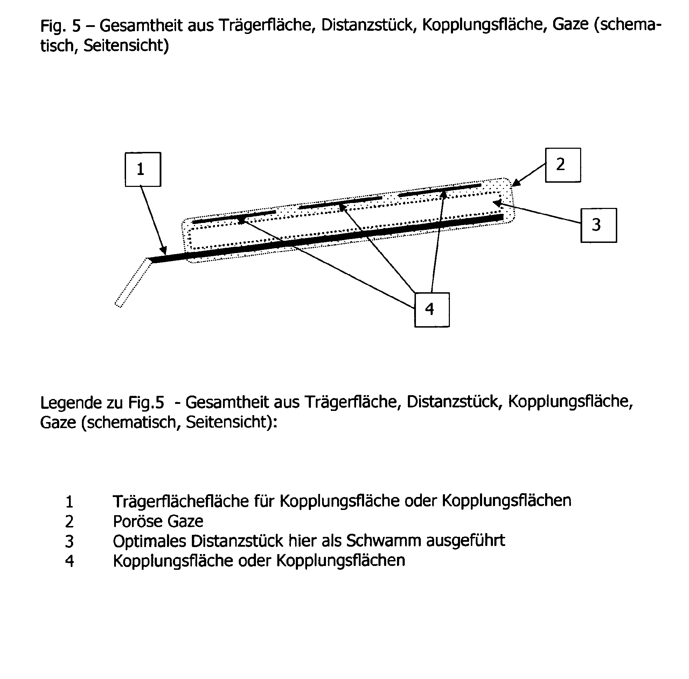

Ferner kann auf der Trägerfläche unter der Kopplungsfläche oder Struktur ein Distanzstück (Fig.5) in Form eines Schwammes oder Gewebes gleich welcher Struktur oder Härte aufgebracht werden. Trägerfläche, Distanzstück, Kopplungsfläche und

Gase können in verschiedenen Materialien, Formen ausgeführt werden. Ohne die Patentansprüche einzuschränken kann als Beispiel die Ausgestaltung der Form in anatomisch der Nasenschleimhaut angepasster Art ausgeführt werden.

Die Gesamtheit aus Trägerfläche mit oder ohne Distanzstück mit Kopplungsfläche oder Struktur mit oder ohne poröser Gase stellt die erfindungsgemäße Vorrichtung dar (Fig.5).

Diese Vorrichtung ist die Basis für das erfindungsgemäße Verfahren der Simulation eines Reaktionswegs und die folgenden analytischen Schritte. Das erfindungsgemäße Verfahren verwendet kovalent oder chelatartig gebundene Stoffe, Substanzen oder Gemische diese werden an die Nasenschleimhaut gebracht indem der Trägerfläche die Kopplungsfläche oder Struktur an die Nasenschleimhaut presst. Damit wird die Exposition der Nasenschleimhaut mit einem Stoff simuliert. Durch die kovalent oder chelatartige Bindung wird verhindert, dass die auf der Kopplungsfläche oder Struktur gebundenen Stoffe, Substanzen oder Gemische in die Nasenschleimhaut und damit in den Körper des Patienten eindringen können.

Als ein Beispiel, welches den Patentumfang nicht einschränkt, sind die allergischen Erkrankungen zu nennen. Die häufigste Erkrankung ist die Rhinitis allergica, deren Morbidität in Mitteleuropa mit 10 bis 20% in der wissenschaftlichen Literatur angegeben wird. Natürlicherweise trifft ein in der Atemluft befindliches Allergen auf die Nasenschleimhaut. Die dort vorhandenen Mastzellen samt IgE Antikörpern und weiteren Immunglobuline erkennen das oder die Allergene. Im positiven Fall wird eine Reaktionskaskade ausgelöst, welche anschließend die Erkrankungs üblichen Symptome der zur Folge hat.

Der erfindungsgemäße Simulator, hat ein oder mehrere Allergene auf der Kopplungsfläche welche Kontakt zur Nasenschleimhaut hat, gebunden. Es wird also die Exposition der Nasenschleimhaut mit einem Allergen und die Bindung des Antikörper bzw. einer Zelle an das Allergen simuliert und damit den der Hauptteil des Beginns einer allergischen Kaskade, die zur Auslösung der Symptome einer allergischen Erkrankung führt.

Die IgE Antikörper oder andere Subklassen der Immunglobuline binden im positiven Falle an die gebundenen Allergene auf der Kopplungsfläche. Da die Antikörper nach der Bindung an das Allergen ebenfalls gebunden sind, können sie nicht mehr das Signal zum Start der Reaktionskaskade auslösen; der Patient bleibt Beschwerde frei und kann nicht immunisiert (allergenisiert) werden. Nach Entfernen der Trägerfläche von der Nasenschleimhaut, samt Kopplungsfläche, kann die Konzentration der gebundenen IgE Antikörper oder anderer Immunsubklassen außerhalb des Körper mit dem Stand der Technik entsprechenden in vitro ELISA Methoden gemessen werden. Die gemessene Konzentration an allergenspezifischen IgE-Antikörper ist ein international akzeptiertes Maß für die Ausprägung einer allergischen Erkrankung vom Typ-1. Da auch Zellen und andere Immunglobulin Subklassen an die Allergene auf der Kopplungsfläche im positiven Fall binden, können auch andere Typen von allergischen Erkrankungen nachgewiesen werden.

Als ein weiteres Beispiel, welches ebenfalls den Patentumfang nicht einschränkt, ist die Verlaufskontrolle von Medikamentengaben bei Patienten. In diesem Fall sind Antikörper gegen die Wirksubstanz des Medikamentes oder und deren Metaboliten an der Kopplungsfläche oder Struktur kovalent gebunden. Wird die Kopplungsfläche in definierten Abständen mittels der erfindungsgemäßen Trägerflächen an die Nasenschleimhaut gebracht, binden die spezifischen Antikörper das Medikament oder und deren Metabolite. Nach entfernen der Kopplungsfläche samt der gebundenen Medikamentmoleküle oder und deren Metabolite können pharmakologische Daten wie Blut- oder Gewebekonzentration zeitlich aufgelöst bestimmt werden, in dem klassische *in vitro* analytische Methoden wie z.B. HPLC oder GC angewendet werden. Damit kann das Anfluten von Medikamenten oder deren Metabolite im Speichergewebe des Körpers wie Fettgewebe oder Muskelgewebe zeitlich aufgelöst simuliert werden.

Das erfindungsgemäße Verfahren stellt die Gesamtheit aus kovalenter oder chelatartiger Bindung eines oder mehrer Stoffe, Substanzen oder Gemische, deren Exposition mittels der erfindungsgemäßen Trägerfläche an der Nasenschleimhaut und die Verbindung mit einem nach dem Stand der Technik vorhandenen analytischen Verfahren dar.

## Patentansprüche

1. Vorrichtung und Verfahren als Simulator benannt **dadurch gekennzeichnet, dass** eine Vorrichtung verwendet wird, die mittels einer Trägerfläche gleich welchen Materials oder Form eine oder mehrere Kopplungsflächen gleich welchen Materials, Form, Dicke an die Nasenschleimhaut für einen endlichen Zeitraum in vivo gepresst, auf der kovalent eine oder mehrere Substanzen gebunden sind, welche durch eine poröse Gaze auf dem Trägerfläche fixiert ist und nachdem zeitlich begrenzten Zeitraum von der Nasenschleimhaut entfernt werden kann, sodass anschließend die Kopplungsfläche vom Trägerfläche entfernt werden kann und mit dieser oder diesen außerhalb der Nasenschleimhaut in vitro analytische Untersuchungen durchgeführt werden können. Ferner das zwischen Kopplungsfläche und Trägerfläche ein Anstandshalter welcher in verschiedene Materialien, Härten, Dicken und Porositäten ausgeführt werden, zwischen gelegt werden kann.

2. Trägerfläche nach Anspruch 1, **dadurch gekennzeichnet, dass** dieser als Klammer mit zwei Schenkeln ausgestaltet ist die nach außen oder nach innen einen Pressdruck auf zwei Seiten der Nasenschleimhaut ausüben können und anatomisch der Nasenschleimhaut angepasst ist.

3. Trägerfläche nach Anspruch 2, **dadurch gekennzeichnet, dass** dieser als Klammer ausgeführt ist, welche einen Trägerfläche an die innen Seite an je einer äußeren Nasenwand fixieren kann.

4. Trägerfläche nach Anspruch 2, **dadurch gekennzeichnet, dass** dieser aus zwei getrennten Teilen aufgebaut ist, wobei ein Teil der Trägerfläche entspricht welche an die innen Seite an einer äußeren Nasenwand mittels magnetischer Kraft fixiert werden kann und die andere Teil den dazu notwendigen Magneten, als Dauermagneten oder Elektromagneten, aufnimmt.

5. Kopplungsfläche nach Anspruch 1, **dadurch gekennzeichnet, dass** diese aus verschiedenen Materialien ausgeführt ist und diese Materialien in verschiedenen Formen, Dicken, Netzen oder Poren ausgeführt werden können.

6. Kopplungsfläche nach Anspruch 1 und 4, **dadurch gekennzeichnet, dass** eine oder mehrere Substanzen, Stoffe oder Gemische einzeln oder von einander getrennt kovalent oder mittels Chelatkomplex gebunden werden.

7. Fixierung der Kopplungsfläche nach Anspruch 1, 4 und 5, **dadurch gekennzeichnet, dass** diese durch eine netzartige poröse Gaze, welche aus verschieden Materialien bestehen kann fixiert wird oder das die Fixierung durch Verklebung, klemmen, nageln oder nieten ausgeführt wird.

8. Abstandshalter nach Anspruch 1 **dadurch gekennzeichnet, dass** dieser als Schwamm oder anderes weiches Material ausgeführt wird.

9. Verfahren nach Anspruch 1 **dadurch gekennzeichnet, dass** eine oder mehrere Kopplungsflächen nach Anspruch 1, 4 und 5 mit einem oder mehreren Allergenen oder -gemischen aus diesen kovalent beschichtet sind und zum Nachweis von allergischen Erkrankungen genutzt werden.

10. Verfahren nach Anspruch 1 **dadurch gekennzeichnet, dass** eine oder mehrere Kopplungsflächen nach Anspruch 1, 4 und 5 mit einem oder mehreren Antikörpern oder Gemischen aus diesen kovalent beschichtet sind und zum Nachweis von Substanzen in der Nasenschleimhaut genutzt werden kann. Ferner das diese auch mit einer zeitlich festgelegten Reihenfolge an die Nasenschleimhaut eingesetzt werden.

11. Verfahren nach Anspruch 1 **dadurch gekennzeichnet, dass** eine oder mehrere Kopplungsflächen nach Anspruch 1, 4 und 5 mit einem oder mehreren Substanzen oder Gemischen aus diesen kovalent beschichtet sind und zum Nachweis von freien körpereigenen Peptiden, RNA, DNA oder anderen bindungsfähigen Substanzen in der Nasenschleimhaut genutzt werden kann. Ferner das diese auch mit einer zeitlich festgelegten Reihenfolge an die Nasenschleimhaut eingesetzt werden.
